# EUROPEAN PATENT SPECIFICATION

(11) **EP 1 698 284 B1**
(45) Date of publication and mention of the grant of the patent: **02.04.2008**
(21) Application number: 06251021.9
(22) Date of filing: 25.02.2006
(51) Int. Cl.: A61B 17/12, A61F 2/01

(54) **Embolic coil with twisted wire**
Emboliespiralfeder aus verdrehtem Draht
Spirale embolique à fil tourné

(30) Priority: 02.03.2005 US 70727
(43) Date of publication of application: 06.09.2006
(73) Proprietor: Cordis Neurovascular, Inc., Miami Lakes, Florida 33014 (US)
(72) Inventor: Farnan, Robert C., Davie, Florida 33328 (US); Lorenzo, Juan A., Davie, Florida 33328 (US); Pomeranz, Mark L., Davie, Florida 33325 (US); Sherman, Darren R., Fort Lauderdale, Florida 33301 (US)
(74) Representative: Belcher, Simon James

(56) References cited:
- DE-B3- 10 257 219
- US-A- 6 156 061
- US-A1- 2003 120 302
- US-B1- 6 338 736

## Description

Vasoocclusive devices are used to occlude a vascular structure in the body, such as an aneurism. Also, blood vessels may need to be occluded, for example, because of cancer, wound, or stroke, for the purpose of avoiding an embolism in the case of a wound or stroke, and for the purpose of depriving blood to a tumor.

Various published references which describe vasoocclusive devices include US-A-2003/0120302 (Minck Jr.); US-6338736 (Boosfeld); Patent Publication US-A-2002/0193819 (Porter); and US-5718711 (Berenstein et al.). Specifically, vasoocclusive devices comprise an embolic element.

It of course is desirable for efficient clotting to be provided by the embolic element, after it has been placed in position within a blood vessel. One way that this can be done is by disrupting flow and causing turbulence, leading to increased clotting.

By this invention, an embolic element is provided which has improved clotting characteristics that appear after the embolic element has been inserted into its desired position within a blood vessel, which is often a medically urgent matter.

According to an aspect of the invention, there is provided an embolic element which comprises a coil of wire. The coil extends along a first longitudinal axis of the coil, individual loops of the coil being generally longitudinally spaced. The wire comprises at least a portion of flat wire having a second wire axis extending longitudinally along the wire, the flat wire being twisted about the second wire axis.

According to another aspect of the invention, there is provided an embolic element which comprises a length of wire. The length of wire, in turn, comprises at least one flat portion which is twisted to define a generally helical shape, thus providing added turbulence to blood flow, as well as having added surface area for stimulating clotting, when compared with round wire. The wire is preferably twisted about its longitudinal axis.

Then, it is preferred for the twisted embolic element also to generally be bent to essentially define a three-dimensional shape, for example a coil that extends along a first longitudinal axis (of the coil), with individual loops of the coil being generally longitudinally spaced along the axis. This coil may be a helical coil if desired, or the individual coil may vary in diameter. Such a coil, comprising at least a portion of flat wire, and, if desired, comprising a continuous, flat ribbon of wire, has a second wire axis extending longitudinally along the wire (which is different from the first longitudinal axis which extends through individual loops of the coil), with the flat wire portions being twisted (as stated above) about the second wire axis.

If desired, the wire used may comprise spaced, tubular wire portions, separated by portions of the flat wire. This may be accomplished by flattening separate portions of a length of tubular wire, so that the flat portions comprise flattened wire tubing, separated by the remaining, unflattened, tubular wire portions. However, it is also possible, as previously stated, for the entire length of wire to be substantially all flat. For example, a single layer ribbon of wire may be used, contrary to the double layer portions of flattened wire tubing, separated by the unflattened, tubular wire portions.

It may be desired for a second wire to be included in the coil of wire described above. This second wire may be of substantially circular, triangular, rectangular, or oval cross section or the like, and may have either a larger or smaller diameter than the spiraled wire having flat portions. One or both of the wires may have a diameter ranging from 0.038 to 0.254 mm (0.0015 to 0.010 inch) in diameter. The two wires may have differing diameters, so that the diameter ratio of the larger diameter to the smaller diameter may be from about 2:1 up to about 10:1 1 in diameter (the flat wire of course does not precisely have a diameter, but its width may serve as the measured "diameter."). The materials of the two wires may be the same, but they may also vary. In some embodiments, at least one of the wires may be made of a radiopaque material such as platinum alloy, tantalum, or the like. (The radiopaque material is preferably more radiopaque than stainless steel.) Either of the wires may be metallic or polymeric.

For example, one or both of the wires may be made of a bioabsorbable polymer, such as PCL/PGA (polycaprolactone/polyglycolide), which material is designed to elicit and increase an inflammatory response in a known manner. This bioabsorbable polymer may be found on the surface of one or both wires, or the wire itself may be made of a plastic comprising the bioabsorbable polymer.

In one embodiment, at least some of the wire may comprise a thrombolytic agent.

Further by this invention, a method is provided for manufacturing an embolic element, which comprises: twisting a length of wire about a longitudinal axis thereof, in which the wire has at least one flat portion along its length, to cause the flat portion to form a first generally helical shape. Then, the length of twisted wire is wound onto a mandrel by bending of the wire to form a three-dimensional shape. This three-dimensional shape is typically a coil, such as another helix, as stated before. This coiled wire may then be used to form other three-dimensional shapes, such as a sphere, or an oval "football" shape, that can pivot flat at ends thereof for insertion to a blood vessel or aneurism. Also, the coiled wire as formed may have generally rectangular loops rather than the cylindrical loops of a typical helix, or other shapes as desired, comprising a three-dimensional structure, typically by bending a length of the twisted wire of this invention on an appropriate mandrel.

As stated before, the twisted wire may be formed from a completely flat ribbon, or one of varying shape, such as a wire of alternating tubular and flattened tubular segments. Three-dimensionally shaped forms made of this twisted wire may comprise substantially a cylinder formed from the wire coil (forming a second, larger helix if desired), or a variety of conical shapes, a sphere, a spheroidal shape, or the like.

Embodiments of the invention will now be described by way of example with reference to the accompanying drawings, in which:
Figure 1 shows a helical embolic element in accordance with this invention;
Figure 2 shows an enlarged, fragmentary view of a portion of the helical element of Fig. 1;
Figure 3 is an enlarged view of a portion of another embodiment of an embolic element, comprising a wire which may be placed in a helical array as illustrated in Fig. 1; and
Figure 4 is a similar, enlarged view of the portion of the embolic element of Fig. 3 prior to twisting of the embolic element.

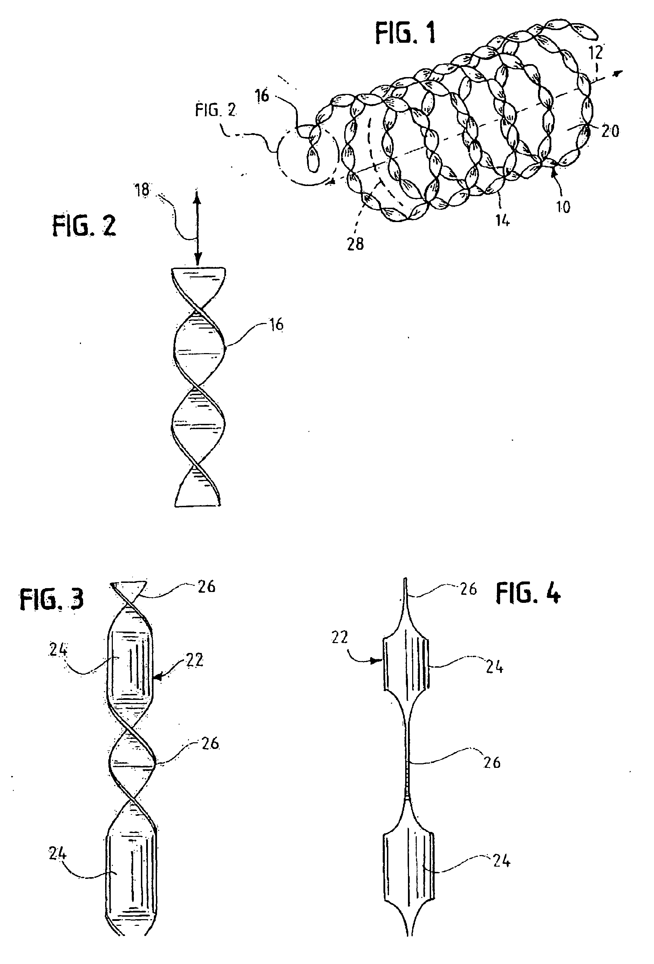

Referring to the drawings, Fig. 1 illustrates a helical, embolic element in accordance with this invention which can be mounted on a catheter in conventional manner and advanced through the vascular system of the patient to a site where clotting or other blockage is medically desirable, to fill an embolism to protect it from rupturing, or to block flow in a blood vessel, as previously described, and as generally known in the art.

Embolic element 10 can be seen to define a coil of wire, specifically a helix, which coil extends about first longitudinal axis 12, the individual loops 14 of the coil being generally longitudinally spaced from each other along axis 12. The structure comprises, in this embodiment, a single, extending length of wire 16. Specifically, as indicated in Fig. 2, the length of wire 16, which comprises embolic element 10 and the helical loops 14, further comprises a wire which is flat like a ribbon along its entire length, and which has been twisted about a second wire axis 18, which extends longitudinally along wire 16, and thus is a different axis from the coil axis 12 discussed above.

As previously stated, such a helically twisted ribbon of wire 16 serves to provide added turbulence to blood flow in its vicinity, when compared with a helically arranged, flat ribbon of wire which is not twisted, and which provides added surface area for stimulating clotting, when compared with a wire of circular cross section. Thus, when twisted wire 16 is formed from a cylindrical mandrel 20 which is of the shape of the interior of helical, embolic element 10, wire 16, being wound upon it, assumes the shape of mandrel 20 to form another, larger helical shape than the helix defined by wire 16 along axis 18.

Additionally, twisted wire 16 may be wound on mandrels of other shapes to form other shaped objects. Also, the helical embolic element 10 may be wound on a mandrel in its own right, and combined with other lengths of helical wire to form a three-dimensional structure such as spherical or oval structure, which may be mounted on a catheter in elongated form, and then released to expand to form the spherical or oval structure in an aneurism, for example, or an artery, to promote clotting and to fill a pre-determined space with clotted material, for known medical benefits.

Referring also to Figs. 3 and 4, the helical, embolic element 10 may comprise a wire of different structure. Specifically, rather than comprising a twisted, flat ribbon 16 as in Fig. 2, wire 22 may comprise spaced, tubular wire portions 24, separated by twisted portions of flat wire 26. As shown in Fig. 4, the portions of flat wire 26 may comprise flattened wire tubing, so that this length of wire 22 which is used to form an embolic element such as helical element 10 may comprise wire tubing, not ribbon as in the embodiment of Fig 2. The tubing is then flattened into a series of double layer, flat sections 26 as shown in Fig. 4, prior to twisting of sections 26 into helical structures as shown in Fig. 3. The tubular wire sections which are between the flattened wire sections 26 then constitute tubular wire portions 24. Then, after formation of the flattened sections 26 in the tubular wire, as shown in Fig. 4, the tubular wires are twisted, resulting in twisting of the flattened wire sections 26 to form generally helical, twisted, flat wire portions 26 as shown in Fig. 3. This length 22 of twisted, flat wire segments may then be formed into another helix 10 on mandrel 20 in a manner similar to the previous embodiment. Such an embolic element 10 may be used in its own right, or it may be formed into a three-dimensional structure such as a sphere or oval (football) shape in the manner described above, for implantation into an aneurism or the like, with twisted portions of the wire providing an improvement in the clotting and other characteristics of the structure.

If desired, as shown in Fig. 1, a second wire 28, for example, a wire of circular or oval cross section, may be incorporated into embolic element coil 10 forming a second, helical array interleaving the helical array of wire 16.

In some embodiments, at least one of wires 16, 28 may be made of a material that is more radiopaque than stainless steel, such as tantalum. Also, in some embodiments at least some of the wire may carry a thrombolytic or other material that elicits and increases an inflammatory response. For example this may be a bioabsorbable polymer, as previously described. Also, part or all of one of the wires 16, 28 may be made of such a thrombolytic agent, bioabsorbable polymer, or the like.

Thus, by this invention, an embolic element may be provided, comprising a length of wire, in which the wire comprises at least one flat portion which is twisted about its longitudinal axis to define a generally helical shape, examples being illustrated in the drawings.

## Claims

1. An embolic element (10) which comprises:
a coil of wire (16), said coil extending along a first longitudinal axis (12) of said coil, individual loops (14) of said coil being generally longitudinally spaced;
said wire (16) comprising at least a portion of flat wire having a second wire axis (18) extending longitudinally along said wire (16), **characterised in that** said flat wire is twisted about said second wire axis (18).

2. The embolic element of claim 1 in which said wire comprises spaced, tubular wire portions (24), separated by twisted portions (26) of said flat wire.

3. The embolic element of claim 2 in which said portions (26) of said flat wire comprise flattened wire tubing.

4. The embolic element of claim 1 in which said wire (16) is substantially all flat.

5. The embolic element of claim 4 in which said flat wire (16) is of a single layer.

6. The embolic element of claim 1 in which said coil of wire (16) is a helical coil.

7. The embolic element of claim 1 in which a second wire (28) is included in said coil of wire (16).

8. The embolic element of claim 7 in which said second wire (28) is of substantially circular cross section.

9. The embolic element of claim 7 in which at least one of said wires (16,28) is made of a material that is more radiopaque than stainless steel.

10. The embolic element of claim 1 in which at least some of said wire (16) carries a thrombolytic agent.

## Patentansprüche

1. Embolieelement (10), welches umfaßt:
eine Drahtspule (16), wobei sich die Spule entlang einer ersten Längsachse (12) der Spule erstreckt, wobei einzelne Schleifen (14) der Spule im allgemeinen longitudinal beabstandet sind;
wobei der Draht (16) zumindest einen Teil aus einem flachen Draht mit einer zweiten Drahtachse (18) aufweist, die sich longitudinal entlang des Drahts (16) erstreckt, **dadurch gekennzeichnet, daß** der flache Draht um die zweite Drahtachse (18) gewunden ist.

2. Emobolieelement nach Anspruch 1, bei dem der Draht beabstandete röhrenförmige Drahtabschnitte (24) aufweist, die durch gewundene Abschnitte (26) des flachen Drahts getrennt sind.

3. Emobolieelement nach Anspruch 2, bei dem die Abschnitte (26) des flachen Drahts einen abgeflachten Drahtschlauch aufweisen.

4. Emobolieelement nach Anspruch 1, bei dem der Draht (16) im wesentlichen vollständig flach ist.

5. Emobolieelement nach Anspruch 4, bei dem der flache Draht (16) aus einer einzelnen Lage besteht.

6. Emobolieelement nach Anspruch 1, bei dem die Drahtspule (16) eine spiralförmige Wendel ist.

7. Emobolieelement nach Anspruch 1, bei dem ein zweiter Draht (28) in der Drahtspule (16) umfaßt ist.

8. Emobolieelement nach Anspruch 7, bei dem der zweite Draht (28) einen im wesentlichen kreisförmigen Querschnitt aufweist.

9. Emobolieelement nach Anspruch 7, bei dem zumindest einer der Drähte (16, 28) aus einem Material hergestellt ist, das röntgenstrahlenundurchlässiger ist als nicht rostender Stahl.

10. Emobolieelement nach Anspruch 1, bei dem zumindest ein Teil des Drahtes (16) ein thrombolytisches Agens trägt.

## Revendications

1. Elément embolique (10) qui comprend :
une spirale de fil métallique (16), ladite spirale s'étendant le long d'un premier axe longitudinal (12) de ladite spirale, les boucles individuelles (14) de ladite spirale étant généralement longitudinalement espacées;
ledit fil métallique (16) comprenant au moins une partie d'un fil métallique plat ayant un second axe de fil métallique (18) s'étendant longitudinalement le long dudit fil métallique (16), **caractérisé en ce que** ledit fil métallique plat est torsadé autour dudit second axe de fil métallique (18).

2. Elément embolique selon la revendication 1, dans lequel ledit fil métallique comprend des parties de fil métallique tubulaires et écartées (24), séparées par les parties torsadées (26) dudit fil métallique plat.

3. Elément embolique selon la revendication 2, dans lequel lesdites parties (26) dudit fil métallique plat comprennent une tubulure aplatie de fil métallique.

4. Elément embolique selon la revendication 1, dans lequel ledit fil métallique (16) est substantiellement totalement plat.

5. Elément embolique selon la revendication 4, dans lequel ledit fil métallique plat (16) est en une seule couche.

6. Elément embolique selon la revendication 1, dans lequel ladite spirale de fil métallique (16) est une spirale hélicoïdale.

7. Elément embolique selon la revendication 1, dans lequel un second fil métallique (28) est compris dans ladite spirale de fil métallique (16).

8. Elément embolique selon la revendication 7, dans lequel ledit second fil métallique (28) a une section transversale substantiellement circulaire.

9. Elément embolique selon la revendication 7, dans lequel au moins un desdits fils métalliques (16, 28) est formé par un matériau qui est davantage radio-opaque que l'acier inoxydable.

10. Elément embolique selon la revendication 1, dans lequel au moins une partie dudit fil métallique (16) porte un agent thrombolytique.
